Europäisches Patentamt

European Patent Office

Office européen des brevets

(11) Veröffentlichungsnummer: **0 264 076**
**A2**

(12) **EUROPÄISCHE PATENTANMELDUNG**

(21) Anmeldenummer: **87114765.8**

(22) Anmeldetag: **09.10.87**

(51) Int. Cl.4: **C12N 11/04 , C12N 1/14**

(30) Priorität: **11.10.86 DE 3634761**

(43) Veröffentlichungstag der Anmeldung:
**20.04.88 Patentblatt 88/16**

(84) Benannte Vertragsstaaten:
**AT BE CH DE ES FR GB GR IT LI LU NL SE**

(71) Anmelder: **Hüttermann, Prof. Dr.**
**Forstbotanisches Institut Büsgenweg 2**
**D-3400 Göttingen(DE)**

(72) Erfinder: **Böttcher, Urte**
**Forstbotanisches Institut Büsgenweg 2**
**D-3400 Göttingen(DE)**
Erfinder: **Hüttermann, Alois, Prof. Dr.**
**Forstbotanisches Institut Büsgenweg 2**
**D-3400 Göttingen(DE)**
Erfinder: **Trojanowski, Jerzy, Prof. Dr.**
**Forstbotanisches Institut Büsgenweg 2**
**D-3400 Göttingen(DE)**

(74) Vertreter: **Harders, Gerhard, Dr.**
**Stettiner Strasse 2**
**D-6367 Karben 6(DE)**

(54) **Gestauchte protoplasmareiche Pilzhyphen mit hoher Enzymaktivität sowie Verfahren zu deren Herstellung in grosstechnisch einsetzbarer Form.**

(57) Gestauchte protoplasmareiche Pilzhyphen mit hoher Enzymaktivität, die in großtechnischer Form einsetzbar sind, werden erhalten, indem
    a) die Pilzmycelzellen in Protoplasten überführt werden,
    b) die Protoplasten immobilisiert werden, und
    (c) die Protoplasten in immobilisiertem Zustand zu Pilzhyphen mit gestauchten Zellen regeneriert werden.
Dadurch wird eine Konservierung des aktiven Stadiums, d. h. der Regenerationsphase der Protoplasten möglich. Die regenerierten Protoplasten sind osmotisch stabil und lassen sich dadurch größtechnisch vielseitig verwenden.

EP 0 264 076 A2

## Gestauchte protoplasmareiche Pilzhyphen mit hoher Enzymaktivität sowie Verfahren zu deren Herstellung in großtechnisch einsetzbarer Form

Die vorliegende Erfindung betrifft gestauchte protoplasmareiche Pilzhyphen mit hoher Enzymaktivität, die großtechnisch einsetzbar sind, sowie ein Verfahren zu ihrer Herstellung.

In der DE-PS 30 37 992 ist ein Verfahren zur Herstellung von Bindemitteln für Holzwerkstoffe beschrieben. Dabei werden beispielsweise Sulfitablaugen mit Enzymen versetzt, die aus lignolytischen Mikroorganismen stammen. Zu den lignolytischen Mikroorganismen gehören z.B. die Weißfäulepilze.

Weißfäulepilze, beispielsweise Coriolus versicolor, sind bereits in einer Gelmatrix immobilisiert worden. Ein solches Verfahren, bei dem Calcium-Alginat verwendet wird, ist in Biotchnol. Letters 1981, Bd. 3, Nr. 12, 701-706 beschrieben. Die Immobilisierung hat den Vorteil, daß keine unerwünschte Vermehrung der Biomasse eintritt und daß das immobilisierte Produkt leicht handhabbar ist, beispielsweise leicht aus Lösungen abgetrennt werden kann. Es wurde aber gefunden, daß solche immobilisierten Mycelien eine verminderte Aktivität aufweisen.

Es ist auch bereits bekannt, aus intakten Zellen Protoplasten herzustellen. Protoplasten sind Zellen ohne Zellwände. Intakte Protoplasten sind in isotonischen Medien lebensfähig, sie haben im allgemeinen eine höhere Enzymaktivität als entsprechende Zellen mit Zellwänden. Protoplasten haben jedoch den Nachteil, daß sie osmotisch außerordentlich empfindlich sind. In hypotonischen Medien platzen sie. Ihre großtechnische Anwendung ist daher schwierig.

Aufgabe der vorliegenden Erfindung ist es, Pilzzellen mit hoher Enzymaktivität zu schaffen, die das aktive Stadium des Protoplasten mit der osmotischen Beständigkeit intakter Zellen mit Zellwänden in sich vereinigen.

Gelöst wird diese Aufgabe dadurch, daß die Pilzmycelzellen in Protoplasten überführt werden, die Protoplasten in an sich bekannter Weise immobilisiert werden und die Protoplasten in immobilisiertem Zustand zu Pilzhyphen mit gestauchten Zellen regeneriert werden.

Der Erfindung liegt die überraschende Beobachtung zugrunde, daß Protoplasten, beispielsweise von Weißfäulepilzen, unter definierten Bedingungen die Zellwände regenerieren können, dabei aber über einen längeren Zeitraum das aktive Stadium beibehalten.

Die Protoplasten können nach bekannten Methoden erhalten werden, beispielsweise nach der Methode von Trojanowski, die veröffentlicht ist in Microbios Letters 25, S. 63 - 65 (1984).

Dabei werden Suspensionen der Mycelien mit Enzymen behandelt, die die Zellwände abbauen, beispielsweise mit Novozym aus Trichoderma harzianum oder mit dem bereits im Jahre 1922 von A. Fleming entdeckten Enzym Lysozym. Die Kulturen des Pilzmycels werden in der Enzymlösung suspendiert. Dabei muß die Lösung isotonisch gehalten werden. Unter der Einwirkung des Enzyms bilden sich Protoplasten mit großen Vakuolen mit einem Durchmesser von ca. 20 bis 40 µm.

Die Immobilisierung der gebildeten Protoplasten wird durch ein organisches Gel bewirkt, das für Proteine und Kohlehydrate permeabel sein soll. Die Matrix des Gels soll keinerlei Wechselwirkung mit den enzymatischen Inhaltsstoffen des Protoplasten bewirken, sondern ausschließlich eine vollständige mechanische Umhüllung des Protoplasten mit dem organischen Trägermaterial der Gelmatrix. Die Gelmatrix soll nach Möglichkeit nicht porös sein, um einen Austritt von Protoplastenmaterial zu vermeiden. Als Gelmatrix eignen sich alle der oben charakterisierten organischen Trägermaterialien, die keine toxischen Beeinflussungen des Protoplasten bewirken. Solche Trägermaterialien sind z. B. Alginate, Agar, Carrageenate, Gelatine und dergl. Bevorzugt werden Alginate verwendet, wie beispielsweise Natrium-Alginat.

Die Protoplasten werden möglichst unmittelbar nach ihrer Herstellung mit einer Lösung des Matrixgel-Materials versetzt. Zweckmäßig ist es, die Lösung in kleinen Anteilen in ein Medium einzudosieren, welches die Gelbildung initiiert oder beschleunigt. Bei Verwendung von Na-Alginat hat sich eine Lösung von Calciumchlorid als sehr brauchbar erwiesen. Beim Eindosieren des Matrix-Materials in den Gelbildner wird das Gel in kleinen Kügelchen oder Körnchen gebildet.

Durch die Immobilisierung werden die Protoplasten mit dem Träger zu einer komplexen Einheit verbunden, die sich im Gegensatz zu freiem Material leicht handhaben läßt. Auf diese Weise läßt sich die Biomasse leicht vom Substrat trennen.

Die Regeneration der Zellwände des Protoplasten setzt unmittelbar nach der Immobilisierung ein. Im Mikroskop ist erkennbar, daß die Protoplasten auch nach der Einbindung ihre runde Struktur beibehalten und intakt bleiben. Normale Pilzmycelzellen weisen eine längliche Struktur auf, ihre Länge in der Längsrichtung des Pilzmycelfadens ist größer als ihre senkrecht dazu gemessene Dicke bzw. ihr Durchmesser. Im Längsschnitt gesehen ist ihre Form rechteckig. Protoplasten hingegen nehmen eine kugelige Gestalt an. Diese Form behalten sie auch nach der Einbettung in eine Matrix bei.

Es wurde nun gefunden, daß die eingebetteten Protoplasten auch bei der Regenerierung der Zellwände diese kugelige Form beibehalten und nicht in die länglich gestreckte Form zurückkehren. Sie zeigen also eine neuartige Morphologie, die bislang nicht bekannt war, es handelt sich um morphologisch neue Vertreter bekannter Stämme, die nicht die übliche gestreckte Form aufweisen, sondern ein "gestauchte" Form.

A n verschiedenen Weißfäulepilzen wurde festgestellt, daß sie in dem jungen Stadium der beginnenden Hyphenbildung eine hohe lignolytische Aktivität aufweisen, was durch das Vorhandensein vieler plasmareicher junger Hyphenzellen bedingt ist. Die Aktivität nimmt mit dem Älterwerden der Zelle ab.

Bei den im Gel immobilisierten Protoplasten ist die Regeneration zu Hyphen möglich, gleichzeitig wird eine Zellstreckung, die gleichbedeutend ist mit einer Alterung der Zelle und einen Verlust der biochemischen Aktivität bedeutet, durch die Einbindung in die Matrix mechanisch unterbunden. Damit wird durch die Immobilisierung der Protoplasten eine Konservierung des aktiven Stadiums, d. h. der Regenerationsphase der Protoplasten zu jungem Mycel, über einen längeren Zeitraum möglich. Die regenerierten Zellen können durch Teilung weitere kleine plasmareiche Hyphenzellen bilden.

Durch die Regenerierung der Zellwände werden die Protoplasten beständiger gegenüber osmotischen Einwirkungen, sie müssen dann nicht mehr in einem isotonischen Medium gehalten werden. Da die Enzymaktivität der immobilisierten Protoplasten bis zu einem Zeitraum von mehreren Wochen aufrechterhalten bleibt, werden durch das erfindungsgemäße Herstellungsverfahren großtechnisch einsetzbare Zellen mit hoher Enzymaktivität erhalten.

Die Erfindung wird nachfolgend anhand von Beispielen näher erläutert, die die Durchführung und Wirkungsweise der Erfindung zeigen.

Fig. 1 zeigt die Freisetzungsrate von $^{14}CO_2$ bei Mycel und immobilisiertem Mycel von Heterobasidion annosum;

Fig. 2 zeigt die entsprechende Freisetzungsrate bei Protoplasten und immobilisierten Protoplasten von Heterobasidion annosum;

Fig. 3 zeigt eine Apparatur zur Durchführung des erfindungsgemäßen Verfahrens;

Fig. 4 zeigt den zeitlichen Verlauf der Entfärbung eines Farbstoffs durch Pleurotus florida;

Fig. 5 zeigt freies Mycel von Polyporus pinsitus in 1000facher Vergrößerung, aufgenommen im optischen Mikroskop;

Fig. 6 zeigt immobilisiertes Mycel von Polyporus pinsitus mit gestauchten Zellen in 1000facher Vergrößerung;

Fig. 7 zeigt gestreckte Zellen des freien Mycels von Polyporus pinsitus in 18.000facher Vergrößerung, aufgenommen im Elektronenmikroskop;

Fig. 8 zeigt gestreckte Zellen des freien Mycels von Polyporus pinsitus in 13.500facher Vergrößerung;

Fig. 9 zeigt gestauchte Zellen des immobilisierten Mycels von Polyporus pinsitus in 10.500facher Vergrößerung; und

Fig. 10 zeigt eine Einzelaufnahme einer gestauchten Zelle von Polyporus pinsitus in 34.000facher Vergrößerung;

Fig. 11 zeigt die Häufigkeitsverteilung der Zellängen bei Hyphen aus freiem Mycel und bei Mycel aus immobilisierten Protoplasten von Polyporus pinsitus; und

Fig. 12 zeigt die Häufigkeitsverteilung der Hyphenbreite bei Hyphen aus freiem Mycel und Mycel aus immobilisierten Protoplasten von Polyporus pinsitus.

Beispiel 1

Herstellung der Protoplasten

Aus einem 7 Tage alten Mycel von Heterobasidion annosum wurde ein Inoculum hergestellt, indem das Mycel über ein Nylontuch filtriert wurde und dann mit sterilem Wasser gewaschen wurde. Das isolierte Mycel wurde zweimal mit 0,5 M MgSO₄ gewaschen und das gewaschene Mycel mit einer Lösung von 0,16 % Novozym in 0,5 M MgSO₄ suspendiert. Die Mischung wurde zwei Tage lang unter leichtem Schütteln in sterilisierten Petrischalen inkubiert. Die Lösung wurde durch eine Glasfritte G1 filtriert. Die Bildung der Protoplasten wurde im Phasenkontrastmikroskop beobachtet. Die Susupension wurde unter aseptischen Bedingungen 40 min. bei 2000 g zentrifugiert. Das Protoplasma enthaltende Sediment wurde gesammelt. Die erhaltenen Protoplasten waren sphärische Körper mit grossen Vakuolen mit einem Durchmesser von etwa 20 bis 40 um.

3

Beispiel 2

Immobilisierung

Die Protoplasten wurden unmittelbar nach ihrer Herstellung nach der Methode von Trojanowski in 0,5 M NaCl-Lösung mit einem pH-Wert von 4,5 aufgenommen. 10 ml dieser Suspension wurden mit 10 ml Natrium-Alginatlösung (3 %ig) versetzt, die vorher 20 min bei 121°C autoklaviert worden war. Diese Mischung wurde dann tropfenweise durch eine Kanüle mit einem Durchmesser von 0,8 mm in eine 0,1 M CaCl$_2$-Lösung eingespritzt. Das Gel bildete sich augenblicklich. Nach ca. 2 Std. Ruhe wurden die inzwischen festen Gelkörner mehrmals mit 0,5 M NaCl-Lösung gewaschen.

Zu Vergleichszwecken wurde auch homogenisiertes Mycel immobilisiert.

3 bis 4 Tage alte Kulturen wurden homogenisiert und nach 24 Std. abfiltriert. Das Mycel wurde mindestens einmal mit physiologischer Kochsalzlösung gewaschen. Das Mycelpellet wurde anschließend in 0,9 %iger NaCl-Lösung aufgenommen. 10 ml dieser Lösung wurden mit 10 ml Na-Alginatlösung vermischt und die Mischung in 0,1 M CaCl$_2$-Lösung eingetropft. Die Aufarbeitung erfolgte wie bei den Protoplasten.

Beispiel 3

Inkubationsversuche

Den oben beschriebenen frisch bereiteten Kulturen wird eine definierte Menge eines spezifischen $^{14}$C-markierten Phenols bzw. Dehydrierungspolymerisats (DHP) aus Coniferylalkohol (nach Freudenberg) nach der Aktivitätsbestimmung (in dpm) unter sterilen Bedingungen zugesetzt. Die Bestimmung der $^{14}$CO$_2$-Freisetzung aus den $^{14}$C-markierten Substanzen durch den Organismus im Verlauf der Inkubation erfolgt mit der radiorespiratorischen Methode nach Haider und Trojanowski. Die Kulturen werden bei 25°C inkubiert und dabei mit geringer Geschwindigkeit geschüttelt.

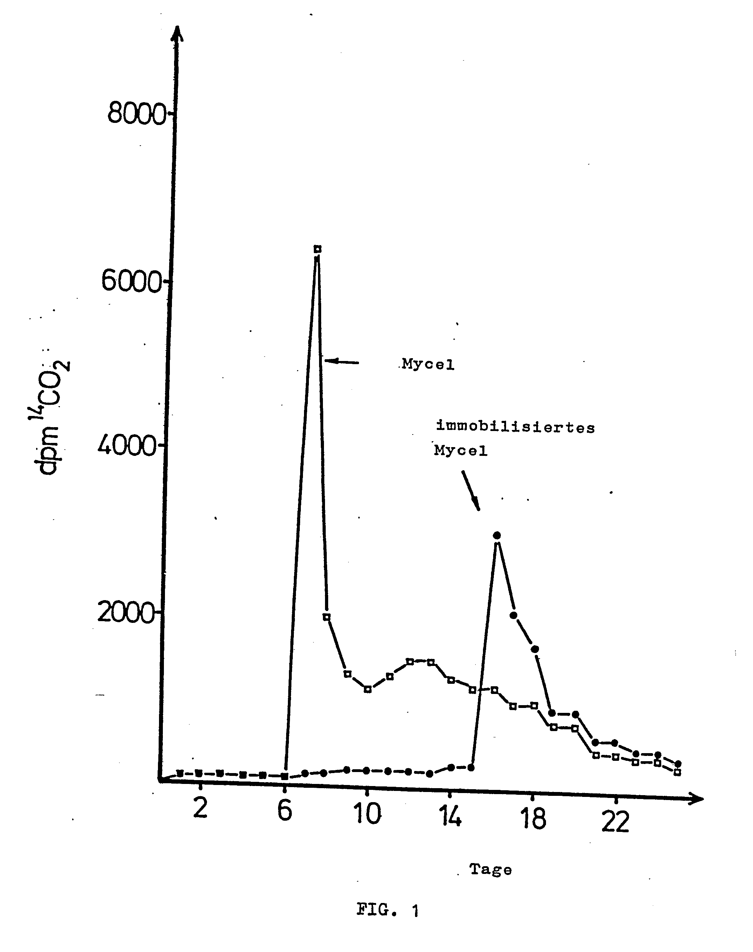

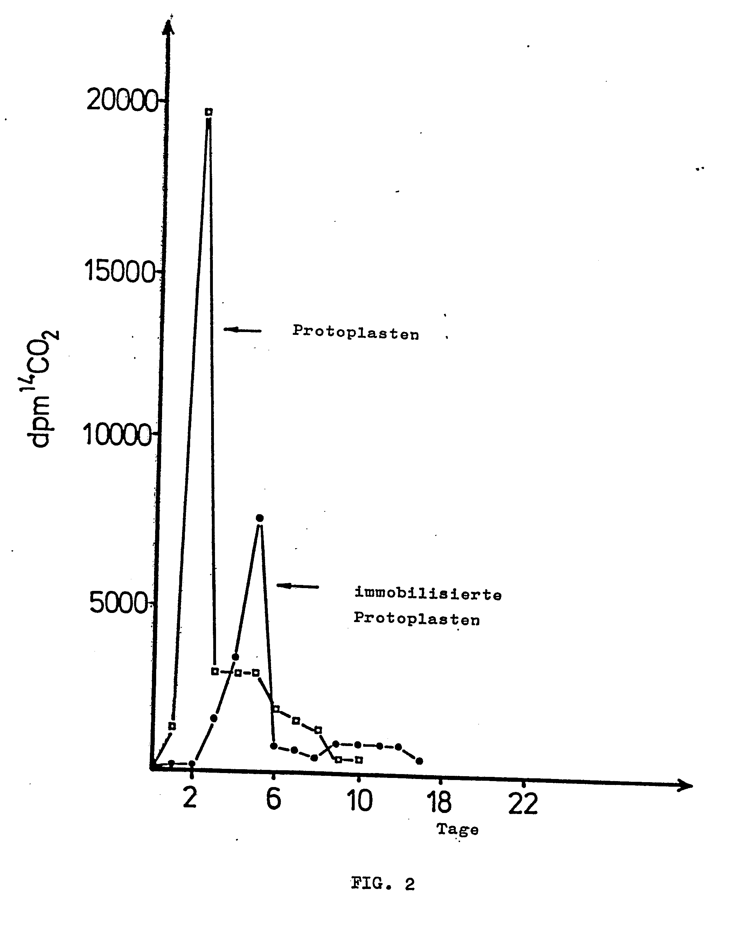

Der Verlauf der $^{14}$CO$_2$-Freisetzung in dpm aus $^{14}$CH$_3$-DHP durch freies und immobilisiertes Mycel sowie durch freie und immobilisierte Protoplasten ist in den Figuren 1 und 2 dargestellt. Auf der Ordinate ist das als $^{14}$CO$_2$ freigesetzte $^{14}$C in dpm pro Kultur bezogen auf die Proteinmenge des Inoculums in mg dargestellt.

Verschiedene Inkubationsversuche mit dem immobilisierten Material in Gegenwart von $^{14}$C-markierten phenolischen Substraten, die als Modellsubstanzen für Lignin und dessen Bausteine fungieren, geben Aufschluß darüber, inwieweit sich die Einbettung auf den Stoffwechsel und die lignolytische Aktivität der Protoplasten bzw. des Mycels auswirkt.

Bei Kulturversuchen mit freiem und immobilisiertem Mycel und immobilisierten Protoplasten von Heterobasidion annosum wurde die $^{14}$C-Freisetzung aus $^{14}$C-markirtem DHP und Monomeren durch Demethylierung, Demethoxylierung, Ringzersetzung, Seitenkettenabspaltung und Decarboxylierung untersucht. Von besonderem Interesse war dabei der Vergleich der Freisetzungsraten von freiem und immobilisiertem Mycel mit denen von immobilis erten Protoplasten. Die ermittelten spezifischen Aktivitäten sind in den Tabellen 1 und 2 zusammengestellt.

Vergleichende Aufstellung der ermittelten spezifischen Aktivitäten beim Abbau verschiedener $^{14}$C-markierter Substrate durch freies und immobilisiertes Mycel und immobilisierte Protoplasten von Heterobasidium annosum und Polyporus pinsitus.

## Tabelle 1

Heterobasidium Annosum

| Substrate | freigesetztes $^{14}CO_2$ (%) pro 10 mg Protein nach 14 Tagen | | |
|---|---|---|---|
| | immob. Protopl. | Mycel frei | Mycel immob. |
| $^{14}C$-Ring-DHP | 0.62 | 0.27 | 0.16 |
| $^{14}C_2$-DHP | 1.58 | 0.43 | 0.19 |
| $^{14}CH_3$-DHP | 2.43 | 1.50 | 0.6 |
| $^{14}CH_3$-Ferula-säure | 7.62 | 2.28 | 0.2 |
| $^{14}COOH$-Vanil-linsäure | 54.55 | 23.6 | 15.02 |
| $^{14}C$-U-Glucose | 56.6 | 17.4 | 20.3 |
| $^{14}C_2$-Ferula-säure* | 6.13 | 5.69 | 0.67 |

## Tabelle 2

Polyporus pinsitus

| Substrate | freigesetztes $^{14}CO_2$ (%) pro 10 mg Protein nach 7 Tagen | |
|---|---|---|
| | immob. Protopl. | freies Mycel |
| $^{14}C$-Ring-DHP | 0.65 | 0.67 |
| $^{14}C_2$-DHP | 1.01 | 0.6 |
| $^{14}CH_3$-DHP* | 2.1 | 0.72 |
| $^{14}CH_3$-Ferulasäure | 2.3 | 1.86 |
| $^{14}COOH$-Vanillinsäure | 47.66 | 16.9 |
| $^{14}C$-U-Glucose | 22.5 | 8.54 |

Mit der Bezugsgröße Protein ist jeweils die Inoculum-Proteinmenge gemeint, die vor der Einbettung bzw. vor dem Ansetzen der Kulturen mit Hilfe eines nach Lowry abgewandelten Proteinnachweises bestimmt wurde.

Die Ergebnisse zeigen, daß die Protoplasten auch nach der Einbettung weiterhin fähig sind, DHP und Monomere durch Ringzersetzung, Demethylierung bzw. Demethoxylierung, Seitenkettenabspaltung und Decarboxylierung zu zerlegen.

Die dabei erzielten Freisetzungsraten sind höher als die von immobilisiertem Mycel und liegen stets in der gleichen Größenordnung wie die des freien Mycels oder darüber.

Immobilisierte Protoplasten von Heterobasidium annosum erreichen also im Vergleich zum Mycel in der Hälfte der Zeit eine gleich große oder sogar höhere quantitative Ausbeute.

Beispiel 4

Anwendungsbeispiel mit Pleurotus florida

5 mg Protein aus Pleurotus florida wurden in 25 ml Suspension aus Na-Alginat immobilisiert und in 250 ml Basidiomycetenmedium vom pH 5 mit folgender Zusammensetzung inkubiert:

Glucose   5 g/l
Hefeextrakt   0,5 g/l
Asparaginsäure   0,65 g/l
$KH_2PO_4$   1,0 g/l
$MgSO_4 \times 7\ H_2O$   0,5 g/l
KCl   0,5 g/l
$FeSO_4 \times 7\ H_2O$   0,01 g/l
$Mn(CH_3COO)_2 \times 4\ H_2O$   0,008 g/l
$Zn(NO_3)_2 \times 4\ H_2O$   0,002 g/l
$Ca(NO_3)_2 \times 4\ H_2O$   0,05 g/l
$CuSO_4 \times 5\ H_2O$   0,003 g/l

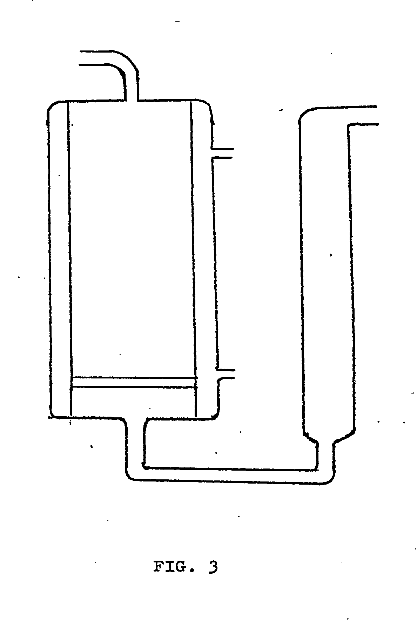

Die Kultur wurde in einem in der Figur 3 gezeigten Reaktor mit Luft begast. Im Medium war ein polymerer Farbstoff gelöst (0,02 % Poly Blue 411, J.K. Glenn und M.M. Gold, 1983). Er wird von den Enzymen entfärbt, die auch für den Ligninabbau verantwortlich sind, und wird deshalb als Indikatorfarbstoff verwendet.

Die Entfärbung wird anhand der Abnahme des Quotienten aus

$$\frac{E_{592}}{E_{482}}$$

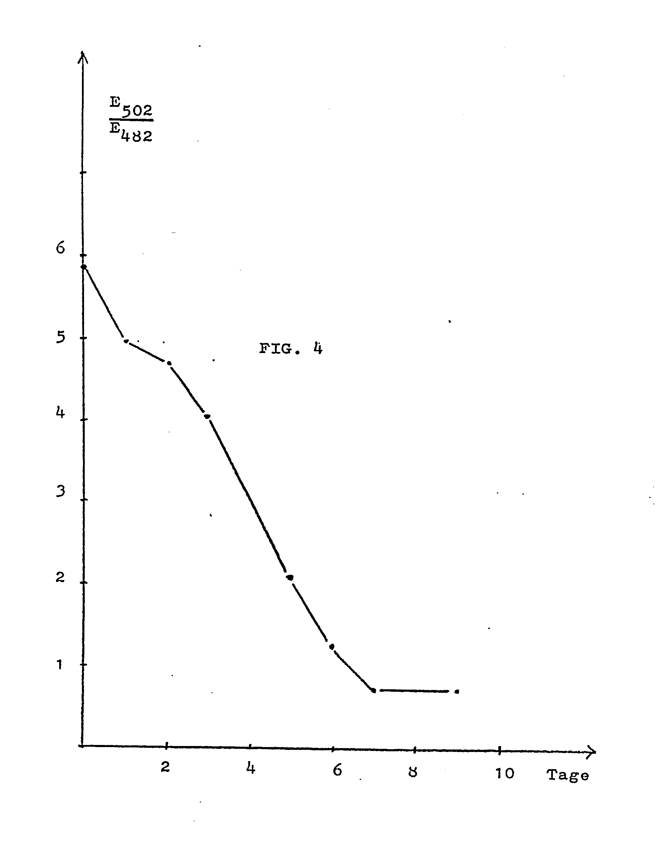

bestimmt. Es wurde eine vollständige Entfärbung innerhalb der ersten 7 Tage festgestellt (s. Fig. 4).
Das immobilisierte Material kann somit phenolische Polymere bzw. Monomere abbauen.

Beispiel 5

Anwendungsbeispiel mit Polyporus pinsitus

Freie Mycelzellen und immobilisierte Protoplasten von Polyporus pinsitus wurden auf folgende Weise erhalten:

Das Pilzmycel wurde homogenisiert und jeweils 10 ml Mycelsuspension auf 50 ml Nährlösung in Roux-Kolben überführt. Nach 24stündiger Inkubation im Brutschrank erfolgte die Ernte des Mycels über einen mit Nylongaze ausgekleideten Büchnertrichter. Dieses wure gründlich mit 0,5 M $MgSO_4$-Lösung gewaschen und zwei Tage mit50 50 ml Novozymlösung in einer großen Petrischale (Durchmesser 20 cm) auf dem Schüttler inkubiert. Die Novozymlösung bestand aus 1,6 mg Novozym 234 (extrazellulärer Enzymkomplex aus Trichoderma harzianum) pro 1 ml 0,5 M $MgSO_4$-Lösung (pH 4,5) und wurde nach Sterilfiltration mit einem 0,2 um Milliporfilter von Sartorius, Göttingen eingesetzt.

Im Anschluß wurden die lysierten Hyphen mit einer Spritze mit einem Kanülendurchmesser von 1,5 mm durch eine Gl Glasfritte gedrückt. Das Filtrat enthielt die gereinigten Protoplasten.

Alle Arbeiten erfolgten unter sterilen Bedingungen.

Immobilisierung der Protoplasten

Das Protoplastenfiltrat wurde zunächst 40 bis 50 Minuten bei 2500 Upm abzentrifugiert. Das erhaltene Pellet wurde in 0,5 M NaCl-Lösung (pH 4,5) resuspendiert, abermals abzentrifugiert (2500 Upm, 35 min) und in 0,5 M NaCl-Lösung aufgenommen. Von dieser Suspension wurden 10 ml (2-4 mg Protein) entnommen und mit 10 ml einer 3 %igen, wässrigen Na-Alginatlösung, die zuvor 15 Minuten bei 121 °C autoklaviert worden war, versetzt und gut gemischt.

Diese Mischung wurde durch eine sterile Kanüle mit 0,8 mm Durchmesser in 0.1 M CaCl₂-Lösung (jeweils ca. 70 ml vorgelegt) getropft. Dabei bildeten sich augenblicklich die Gelkugeln. Ein bis zwei Stunden nach der Herstellung wurden die inzwischen festen Gelkörner mehrmals mit 0,5 M NaCl-Lösung gespült und in das zu untersuchende Substrat überführt. Die Durchführung erfolgte unter sterilen Bedingungen.

Immobilisierung von Mycel

Drei bis vier Tage alte Stammkulturen von Heterobasidion annosum oder Polyporus pinsitus wurden homogenisiert, nach 24 Stunden abzentrifugiert (2500 Upm, 15 min) und mindestens einmal mit physiologischer Kochsalzlösung gewaschen. Das Pellet wurde schließlich in 0,9 %iger NaCl-Lösung aufgenommen. 10 ml (10-14 mg Protein) dieser Suspension wurden mit 10 ml 3 %iger Alginatlösung gemischt und in 0,1 M CaCl₂-Lösung getropft.

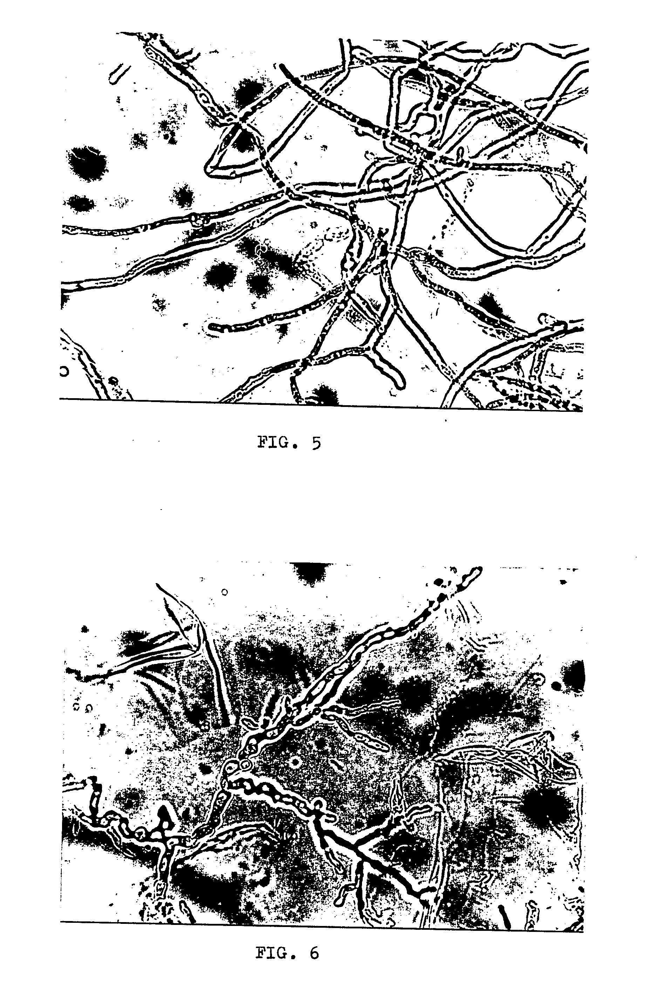

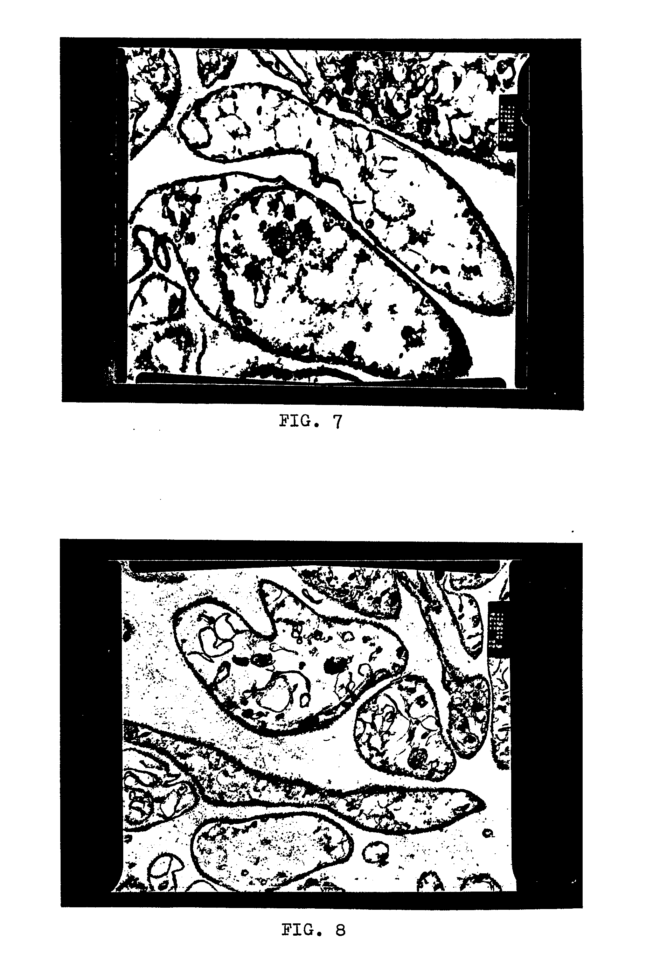

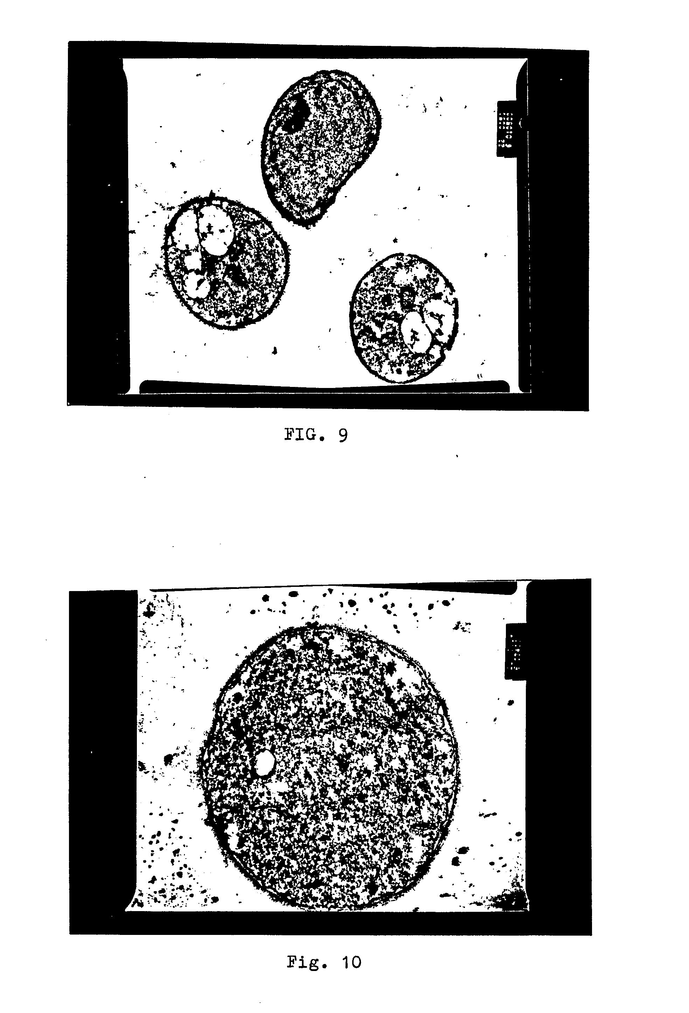

Die Zellformen des freien Mycels sind in den Figuren 5, 7 und 8, die des immobilisierten Mycels in den Figuren 6, 9 und 10 dargestellt. Ein Vergleich der Abbildungen zeigt den Übergang von der gestreckten in die gestauchte Form.

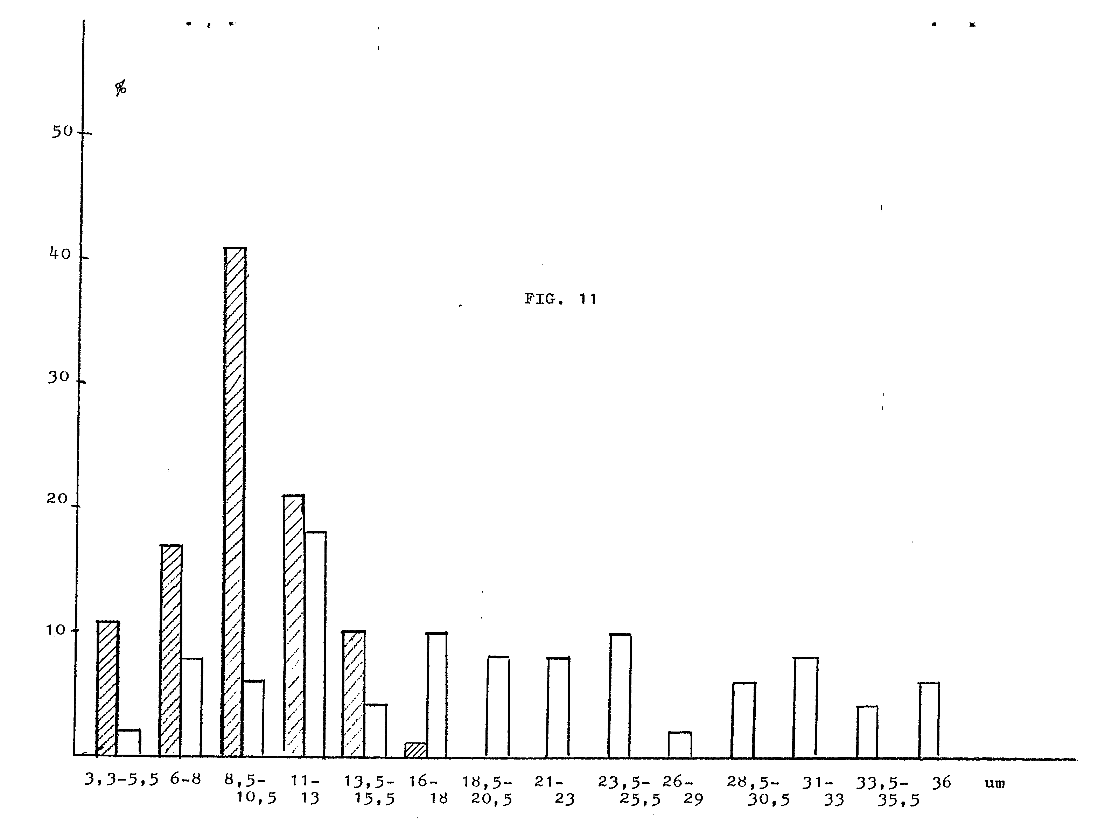

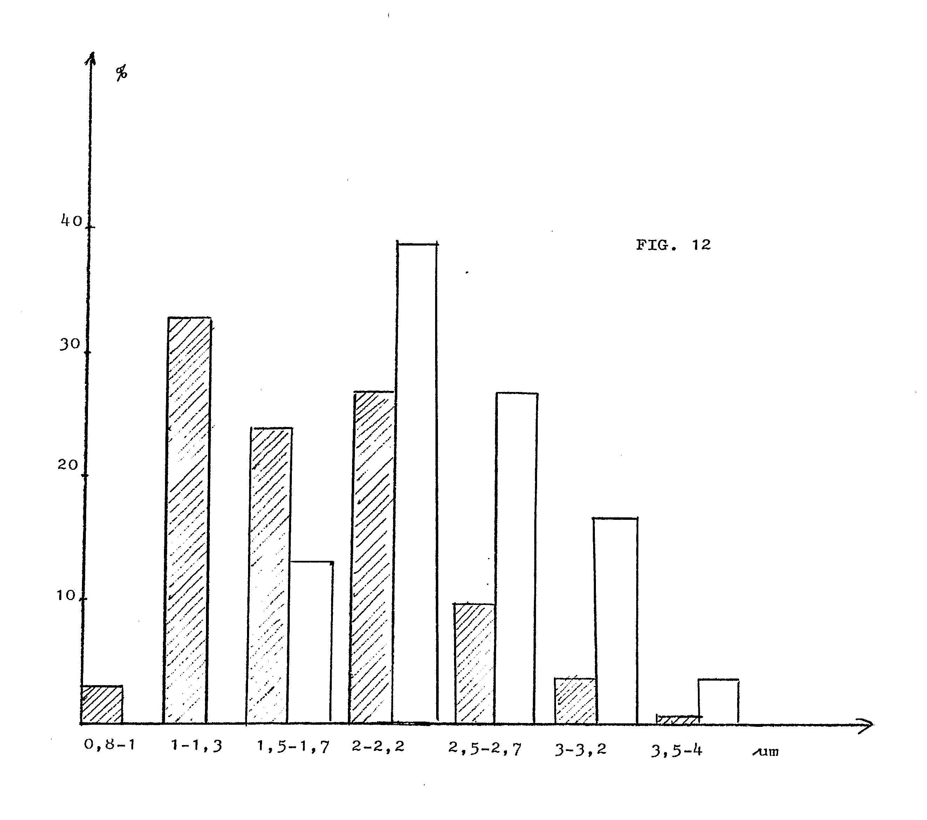

In den Figuren 11 und 12 sind die Häufigkeitsverteilungen der Zellängen und der Hyphenbreite bei Hyphen aus freiem Mycel und bei Mycel aus immobilisierten Protoplasten dargestellt. Durch die Immobilisierung werden die Zellängen beträchtlich verringert. Während bei freiem Mycel die Zellängen bis zu mehr als 36 um betragen können , liegt bei gestauchten Zellen das Maximum der Länge bei 8,5 bis 10,5 um.

Die statistische Auswertung ergibt folgendes:

| Zellänge freies Mycel | Zellänge immobilisiertes Mycel |
|---|---|
| $\overline{M}$ 21,004 um | 8,315 um |
| Standardabweichung: | |
| 12,182 | 3,319 |
| Varianz: | |
| 145,438 | 10,903 |

| Zellbreite freies Mycel | Zellbreite immob. Mycel |
|---|---|
| $\overline{M}$ 2,3 um | 1,717 um |
| Standardabweichung: | |
| 0,522 | 0,61 |
| Varianz | |
| 0,27 | 0,369 |

$$\underline{\text{t-Test:}} \qquad \text{mit } p = 0,05 \qquad 1,96 \text{ x } \frac{\text{Standardabw.}}{\sqrt{n}}$$

$$1,96 \text{ x } \frac{12,182}{\sqrt{50}} = 3,377 \qquad \text{Zellänge freies Mycel}$$

$$1,96 \text{ x } \frac{3,319}{\sqrt{100}} = 0,65 \qquad \text{Zellänge Mycel aus immobil.}$$
$$\text{Protoplasten}$$

---

0,102    Zellbreite freies Mycel

0,12    Zellbreite Mycel ais immobilisierten Protopoasten

Die Grenzen des Konfidenzintervalls:

freies Mycel:    21,004 um - 3,377 um = 17,627 um

imm. Protopl:    8,315 um - 0,65 um = 8,965 um

Daraus ergibt sich ein signifikanter Unterschied der Zellängen bei den beiden verschiedenen Mycelproben.

**Ansprüche**

1. Verfahren zur Herstellung gestauchter protoplasmareicher großtechnisch einsetzbarer Pilzhyphen mit hoher Enyzmaktivität,
**dadurch gekennzeichnet, daß**

    a) die Pilzmycelzellen in Protoplasten überführt werden,

    b) die Protoplasten immobilisiert werden, und

    c) die Protoplasten in immobilisiertem Zustand zu Pilzhyphen mit gestauchten Zellen regeneriert werden.

2. Verfahren zur Konservierung des aktiven Stadiums von Pilzmycel-Protoplasten,
**dadurch gekennzeichnet, daß**

    a) die Protoplasten immobilisiert werden, und

    b) die Protoplasten im immobilisierten Zustand zu Pilzhyphen mit gestauchten Zellen regeneriert werden.

3. Verfahren nach den Ansprüchen 1 oder 2, dadurch gekennze-ichnet, daß
lignolytische Pilze eingesetzt werden.

4. Verfahren nach Anspruch 3, dadurch gekennzeichnet, daß lignolytische Basidiomyceten eingesetzt werden.

5. Verfahren nach Anspruch 3, dadurch gekennzeichnet, daß die Stämme Heterobasidion annosum, Pleurotus florida, Polyporus pinsitus, Phanaerochaete chrysosporium, Sporotrichum pulverulentum oder Cenococcum geophilum eingesetzt werden.

6. Gestauchte protoplasmareiche Pilzhyphenzellen mit hoher Enzymaktivität.

7. Plasmareiche Pilzhyphenzellen mit hoher Enzymaktivität, dadurch gekennzeichnet, daß
sie in gestauchter Form vorliegen.

8. Plasmareiche Pilzhyphenzellen mit hoher Enzymaktivität, dadurch gekennzeichnet, daß
sie in gestauchter Form vorliegen und den Stämmen Heterobasidion annosum, Pleurotus florida, Polyporus pinsitus, Phanaerochaete chrysosporium, Sporotrichum pulverulantum oder Cenococcus geophilum angehören.

FIG. 1

FIG. 2

FIG. 3

$$\frac{E_{502}}{E_{482}}$$

FIG. 4

Tage

FIG. 5

FIG. 6

FIG. 7

FIG. 8

FIG. 9

Fig. 10

FIG. 11

FIG. 12